Europäisches Patentamt

European Patent Office

Office européen des brevets

(11) Publication number: **0 206 469**
**A1**

# (12) EUROPEAN PATENT APPLICATION

(21) Application number: **86303020.1**

(22) Date of filing: **22.04.86**

(51) Int. Cl.⁴: **C 12 M 1/04**

(30) Priority: **24.04.85 GB 8510386**

(43) Date of publication of application: **30.12.86**
**Bulletin 86/52**

(84) Designated Contracting States: **CH DE FR IT LI SE**

(71) Applicant: **Don Whitley Scientific Limited, 14 Otley Road, Shipley West Yorkshire, BD17 7SE (GB)**

(72) Inventor: **Whitley, Donald Charles, "Fairmount" Woodville Road, Keighley West Yorkshire, BD20 6JB (GB)**

(74) Representative: **Cardwell, Stuart Martin et al, Roystons Tower Building Water Street, Liverpool, Merseyside L3 1BA (GB)**

(54) Method and apparatus for establishing and utilising anaerobic conditions.

(57) An anaerobic station and a method of establishing and utilising anaerobic conditions in such a station is described.

In order to provide a more efficient and economic use of the gases required for anaerobic conditions, the anaerobic station is divided into two chambers; a first chamber (12) with an anaerobically maintained atmosphere, which chamber can afford compact storage for specimens being cultured, and a low-oxygen second chamber (14) serving as a manipulation chamber with an otherwise inert gas atmosphere maintained therein. A selective communication (16) is provided between the first and second chambers.

The chamber (14) is provided with a further selective communication (38) to an entry/exit lock (40) which is subjected, when used for access from outside, to inert gas flushing, from the chamber (14). The lock (40) is provided with a displaceable extraction fan (44) to reduce pressure in the lock prior to opening the further selective communication (38).

The pressure in the two chambers is preferably above atmospheric with the pressure in the chamber (12) higher than that in the chamber (14).

ACTORUM AG

Title:   Method and apparatus for establishing and
         utilising anaerobic conditions

DESCRIPTION

The invention relates to a method and apparatus
for establishing and utilising anaerobic conditions,
typically in relation to requirements for chambers
used for culturing micro-organisms requiring absence of
oxygen, in order to grow.

Such requirements arise in microbiology, e.g.
pathology, laboratories in the process of aiding
identification of certain types of bacteria including
disease-producing bacteria.  Conventionally, a
typical anaerobic atmosphere comprises a mixture of
oxygen-consuming gas (hydrogen), an anerobe-nurture
gas (carbon dioxide) and an inert gas (nitrogen),
typically in ratios by volume of 1:1:8, respectively.
At least in relatively advanced countries, such
anaerobic gas mixture is available as such, i.e. already
admixed cylinders, but is not cheap and there is
advantage to be gained from increased efficiency in
its use.   It is, in fact, the case that we have
already made proposals in our European patent application no. 0168915 for economy by using separate supplies of those constituent gases effectively with injection of oxygen-consuming gas only after flushing without that gas. It is an object of this invention to make yet further economy whether, as will be specifically described below, by using pre-mixed anaerobic gas, or by using separate supplies of constituent gases.

To that end, we have devised a radical departure from customary anaerobic chamber design hitherto. Such design has generally been for a relatively large main chamber having an incubated anaerobic atmosphere that is used for specimen culturing with time and for manipulation including opening, inoculation and examination, conveniently using hands-on facilities by way of gloved ports, and for a much smaller lock chamber via which materials are entered and removed, the lock chamber and the main chamber having an interconnecting part that can be opened and closed via the hands-on facility Customarily, the lock chamber is flushed and purged with anaerobic gas mixture at each opening to the outside, or can be so according to our above-mentioned patent application.

According to this invention an anaerobic station comprises a first chamber with an anaerobically maintained atmosphere, which chamber can afford compact storage for specimens being cultured and which may be heated; a low-oxygen second chamber with an otherwise inert gas atmosphere maintained therein and serving for manipulation purposes; and selective communication between those chambers for culturing and manipulation, respectively. It is convenient for both of the first and second chambers to have hands-on facilities via gloved ports.

By manipulation purposes we mean operations including for example loading/unloading of the first chamber and the inspection of specimens etc. It is convenient to refer to the first and second chambers respectively as culturing and manipulation chambers.

According to another aspect of the invention then there is provided a method of establishing and utilising anaerobic conditions in an anaerobic station, the method comprising maintaining a substantially anaerobic atmosphere in a first chamber serving as a storage and culturing chamber, and maintaining a low oxygen otherwise inert gas atmosphere in a second chamber having selective communication with the first chamber, so that loading, unloading inspection and manipulation is done via or in the second chamber.

It is preferred for the manipulation chamber to have further selective communication to an entry/exit lock that is subjected, when used for access from outside, to inert gas flushing, advantageously from the manipulation chamber, i.e. with the aforesaid further selective communication open.   A suitable flushed lock comprises an external tubular extension from that further selective communication that may have a displaceable extraction fan at its otherwise free end.

In operation of the flushed lock, it can be loaded with sealed specimens, say by temporary, displacement of the extraction fan, before opening the further selective communication.   Prior to opening thereof a reduced pressure can be pulled on that lock, say from action of the aforesaid extraction fan.   Then, at opening the further selective communication, the low-oxygen inert gas atmosphere of the manipulation chamber will flood into and flush through the lock, conveniently as drawn by said extraction fan, and is fed to the manipulation chamber at over pressure from compressed source.   After transfer of the specimens into the manipulation chamber, using the hands-on facility thereof, the further selective communication is closed and the extraction fan turned off.

It will be appreciated that opening and inoculation of those specimens in the low-oxygen predominantly inert gas atmosphere of the manipulation chamber can be done quickly for transfer into the culturing chamber without deleterious effect as the anaerobic atmosphere of the latter can be assured to have sufficient oxygen burning gas component to kill off any oxygen entrant thereto. Similar considerations apply to inspection in the manipulation chamber of specimens taken from the culturing chamber, though at least one specimen at a time can usually be first inspected therein, i.e. before transfer of batches to the manipulation chamber.

Specific implementation of the invention will now be described, by way of example, with reference to the accompanying drawings, in which:

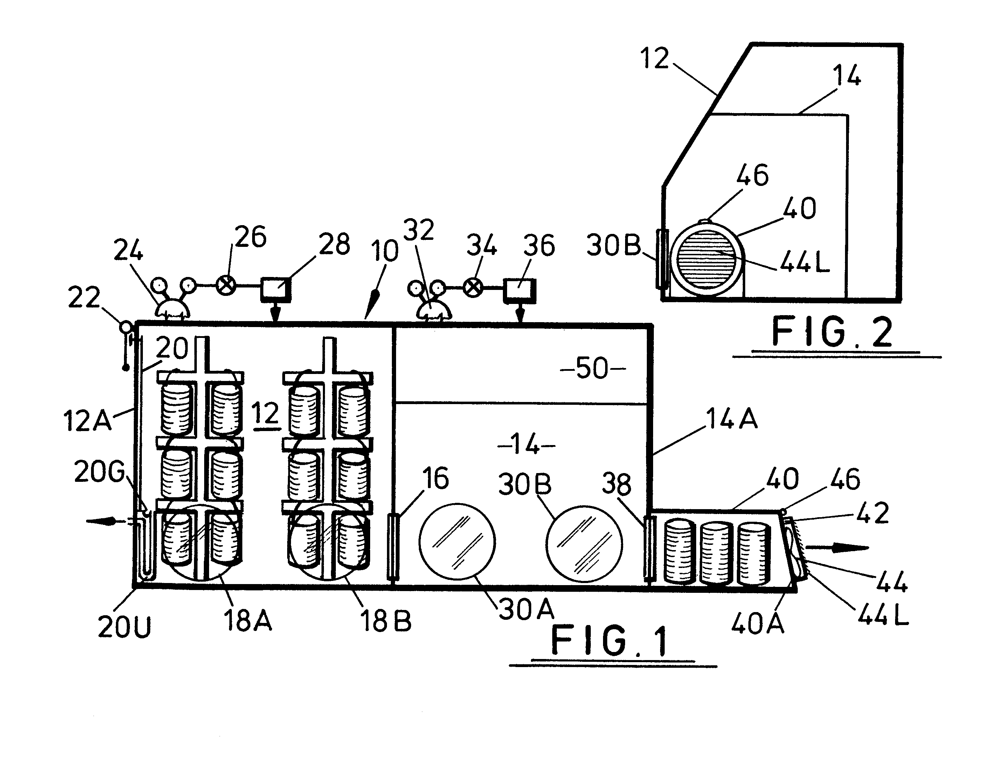

Figure 1 is a schematic elevational view of an anaerobic station;

Figure 2 is a corresponding schematic end view.

In the drawing, anaerobic station 10 comprises a culturing chamber 12, and a manipulation chamber 14 with a selective communication 16 therebetween of the well-known port-with-removable sealing bung type.

The culturing chamber 12 is shown equipped with hands-on facility via front ports 18A, 18B for long gauntlets sealed thereto. Internally, the culturing chamber 12 is shown equipped with carousels or shelves for compact storage of petri dish stacks therein. Also indicated is a dehumidifying condenser plate 20 at its end wall 12A, and which is preferably of air-cooled type with drip gutter 20G and U-type tube drain 20U (providing a water trap). An external cooler blind 22 is indicated for progressively adjustable insulation of the plate 20 from the effects of external lower temperatures. Heating is conveniently by way of electric lamps (not shown) also serving lighting purposes and preferably run off a lower voltage supply than normally intended. A conventional capillary thermostat (not shown) may be used in temperature control. Supply of anaerobic gas mixture is indicated from pressurised cylinder 24 via a solenoid valve 26 and pressure regulator 28 controlling the valve via an associated switch (not shown) operable to maintain a desired over-pressure, for which 1-2 inches (25-50 mm) of water gauge has been found to be satisfactory. Also, the culturing chamber 12 will usually have a circulation fan (not shown) for its anaerobic atmosphere, and a catalyst for oxygen-consuming action at a convenient place in that circulation.

The manipulation chamber 14 is also shown equipped with a hands-on facility via front ports 30A, 30B also for long gauntlets sealed thereto. A nitrogen supply thereto is indicated via pressurised cylinder 32, a solenoid valve 34 and another pressure regulator 36 with associated valve control switch operable to maintain a suitable over pressure in the manipulation chamber 14, but further preferably less than the over-pressure for the culturing chamber 12 and typically $\frac{1}{2}$-1 inch (12.5-25 mm) water gauge. Thus, the manipulation chamber has a low oxygen, predominently inert gas (nitrogen) atmosphere.

At the other end 14A of the manipulation chamber is a further selective communication 38, again conveniently of the port-with-removable sealing bung type, this time to a small lock 40 shown as a tubular extension away from the end 14A. The lock 40 has an inverted U-section and is shown with capacity for three stacks of petri dishes. Its free end 40A is shown closed off by a plate 42 carrying an extraction fan 44 with normally closed louvres 44L and hinged to the lock 40 at 46.

Controls can be located in an instrument panel 50 conveniently located above the manipulation chamber 14. The manipulation chamber 14 serves for inoculation and examination of cultures.

In operation of the flushed lock 40, it can be loaded with sealed specimens by temporary displacement of the extraction fan 44 before opening the further selective communication 38. Prior to opening of the latter, a reduced pressure is pulled on the lock 40 by the action of the aforesaid extraction fan 44. Then at opening of the further selective communication 38, the low-oxygen inert gas atmosphere of the manipulation chamber 14 floods into and flushes through the lock 40. The fan 44 aids this flushing.

The specimens can then be transferred into the manipulation chamber 14 using the hands-on facility thereof, whereafter the further selective communication is closed and the extraction fan turned off.

Opening and inoculation of the specimens in the low-oxygen predominantly inert gas atmosphere of the manipulation chamber 14 can be done quickly for transfer into the culturing chamber 12.

It will be apparent that the apparatus described can accomplish the procedures mentioned above, including control on a suitable basis, and the use of anaerobic gas mixture in the culture chamber 12 is minimal.
Moreover, the nitrogen for the manipulation chamber 14
and flushing purposes can be inexpensive commercial
grades, typically using about 30 litres only for each
lock flushing.

CLAIMS

1. An anaerobic station comprising a first chamber (12) with means for maintaining a substantially anaerobic atmosphere for affording compact storage for specimens being cultured; and characterised by a second chamber (14) with means for maintaining a low oxygen otherwise inert gas atmosphere and serving for manipulation purposes; and selective communication (16) between those chambers for culturing and manipulation respectively.

2. An anaerobic station as claimed in claim 1 in which one or both of the first and second chambers (12, 14) have hands-on facilities via gloved ports (18, 30).

3. An anaerobic station as claimed in any one of claims 1 or 2 in which the second chamber (14) has a further selective communication (38) to an entry/exit lock (40) that is subjected, when used for access from outside, to inert gas flushing.

4. An anaerobic station as claimed in claim 3 in which the flushed lock (40) comprises an external tubular extension from the further selective communication (38).

5. An anaerobic station as claimed in claim 4 in which the tubular extension has a displaceable extractor fan (44) at its free end.

6. A method of establishing and utilising anaerobic conditions in an anaerobic station, the method comprising maintaining a substantially anaerobic atmosphere in a first chamber serving as a storage and culturing chamber, and characterised by maintaining a low oxygen otherwise inert gas atmosphere in a second chamber having selective communication with the first chamber, so that loading, unloading, inspection and manipulation is done via or in the second chamber (14).

7. A method as claimed in claim 6 in which an over pressure is maintained in each of the first and second chambers (12, 14), with the pressure in the first chamber (12) being above that of the second chamber (14).

8. A method as claimed in claim 6 or 7 further comprising, when the second chamber has a further selective communication (38) to an entry/exit lock (40), subjecting the lock, when used for access from outside, to inert gas flushing.

9. A method as claimed in claim 8 in which the lock (40) is flushed from the second chamber (14).

10. A method as claimed in claim 8 or 9 further comprising drawing gas through the lock (40) by means of a displaceable extraction fan (44).

FIG. 1

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 262 091 (M.E. COX)<br><br>* Column 2, lines 34-64; column 3, lines 21-56; column 3, lines 57-68; column 4, lines 34-66; column 4, line 67 - column 5, line 14 * | 1-3,6, 8 | C 12 M 1/04 |
| Y |  | 1-4,6, 8 |  |
| A | FR-A-2 254 409 (M.E. COX)<br>* Figures 1,7; page 8, line 15 - page 10, line 7 * | 3,8 |  |
| Y | US-A-4 111 753 (M.H. FOLSOM at al.)<br>* Figures; claims; column 7, lines 5-60 * | 1-4,6, 8 | TECHNICAL FIELDS SEARCHED (Int. Cl.4) |
|  |  |  | C 12 M<br>B 01 L |

The present search report has been drawn up for all claims

| Place of search | Date of completion of the search | Examiner |
|---|---|---|
| THE HAGUE | 26-08-1986 | COUCKE A.O.M. |